Europäisches Patentamt

European Patent Office

Office européen des brevets

(19)

(11) Veröffentlichungsnummer : **0 185 253
B1**

# EUROPÄISCHE PATENTSCHRIFT

(12)

(45) Veröffentlichungstag der Patentschrift :
27.07.88

(51) Int. Cl.⁴ : **C 07 C   7/04, F 25 J   3/08**

(21) Anmeldenummer : **85115408.8**

(22) Anmeldetag : **04.12.85**

(54) Verfahren und Vorrichtung zur Gewinnung von C2+- oder von C3+-Kohlenwasserstoffen.

(30) Priorität : **17.12.84 DE 3445962
29.03.85 DE 3511636**

(43) Veröffentlichungstag der Anmeldung :
**25.06.86 Patentblatt 86/26**

(45) Bekanntmachung des Hinweises auf die Patenterteilung : **27.07.88 Patentblatt 88/30**

(84) Benannte Vertragsstaaten :
**DE FR GB IT NL**

(56) Entgegenhaltungen :
**DE-A- 3 408 760
GB-A- 2 102 931**

(73) Patentinhaber : **Linde Aktiengesellschaft
Abraham-Lincoln-Strasse 21
D-6200 Wiesbaden (DE)**

(72) Erfinder : **Bauer, Heinz, Dr. rer. nat.
Nibelungenstrasse 6
D-8000 München 19 (DE)**

(74) Vertreter : **Schaefer, Gerhard, Dr.
Linde Aktiengesellschaft Zentrale Patentabteilung
D-8023 Höllriegelskreuth (DE)**

## Beschreibung

Die Erfindung betrifft ein Verfahren zur Abtrennung von $C_{2+}$- oder von $C_{3+}$-Kohlenwasserstoffen aus einem leichte Kohlenwasserstoffe und gegebenenfalls leichter als Methan siedende Komponenten enthaltenden Gasstrom, bei dem der unter erhöhtem Druck stehende Gasstrom abgekühlt, partiell kondensiert und in eine flüssige und eine gasförmige Fraktion getrennt wird und bei dem die flüssige Fraktion durch Rektifikation in einen im wesentlichen $C_{2+}$- bzw. $C_{3+}$-Kohlenwasserstoffe enthaltenden Produktstrom und einen überwiegend leichter siedende Komponenten enthaltenden Restgasstrom zerlegt wird.

Derartige Verfahren dienen vor allem zur Abtrennung von Ethan oder von Propan aus Erdgasen oder anderen Gasen, beispielsweise Raffinerieabgasen. Außerdem eignen sich diese Verfahren zur Abtrennung von vergleichbaren ungesättigten Kohlenwasserstoffen, also beispielsweise von Äthylen oder Propylen, sofern diese Komponenten im zu zerlegenden Gasstrom enthalten sind, was beispielsweise bei Raffinerieabgasen der Fall sein kann. Die Weiterverarbeitung von Raffinerieabgasen ist in letzter Zeit wirtschaftlich interessant geworden, da die Marktpreise für LPG ($C_3/C_4$-Kohlenwasserstoffgemische) gestiegen sind, während umgekehrt Vakuumrückstände sowie Schweröl schlecht verkäuflich sind. Aus diesem Grund werden schlecht vermarktbare schwere Produkte zur Deckung des internen Brennstoffbedarfs einer Raffinerie verfeuert, während gut verkäufliche $C_{2+}$- bzw. $C_{3+}$-Kohlenwasserstoffe aus dem Abgas, das insbesondere bei der Verarbeitung von leichten Rohölbestandteilen zu Benzin in großen Mengen anfällt, abgetrennt werden.

In der älteren, nicht vorveröffentlichten deutschen Patentanmeldung P 34 08 760.5 ist bereits ein Verfahren der eingangs genannten Art beschrieben, das sich auf die Abtrennung von $C_{3+}$-Kohlenwasserstoffen bezieht. Ein wesentliches Merkmal dieser älteren Anmeldung ist darin zu sehen, daß die abzutrennenden $C_{3+}$-Kohlenwasserstoffe bereits während der partiellen Kondensation soweit auskondensieren, daß lediglich das Kondensat in die Rektifikation geführt zu werden braucht, während die nicht kondensierten Anteile nur noch so wenig $C_{3+}$-Kohlenwasserstoffe enthalten, daß auf deren weitere Abtrennung verzichtet werden kann. Die nicht kondensierten Anteile können daher unmittelbar wieder angewärmt und als Restgas abgezogen werden, ohne daß sie vorher die Rektifikation durchlaufen. Das führt zu günstigeren Rektifikationsbedingungen, da insbesondere die Kopftemperatur höher gewählt werden kann.

Aus der GB-A-2.102.931 ist ebenfalls ein Verfahren zur Abtrennung von $C_{2+}$- bzw. $C_{3+}$-Kohlenwasserstoffen bekannt. Der Rohgasstrom wird dort durch stufenweise partielle Kondensation in Abscheidern zerlegt. Die hierbei anfallenden Kondensate werden einer Rektifiziersäule zugeführt, in welcher die $C_{2+}$- bzw. $C_{3+}$-Abtrennung stattfindet. Das $C_{2+}$- bzw. $C_{3+}$-Produkt wird am Sumpf der Rektifiziersäule abgezogen, während die über Kopf anfallende Restgasfraktion erneut in den Prozeß der stufenweise partiellen Kondensation zurückgeführt wird. Die im letzen Abscheider anfallende, gasförmig verbliebene Fraktion wird aus dem System abgezogen.

Der Erfindung liegt die Aufgabe zugrunde, ein Verfahren der eingangs genannten Art weiter zu verbessern, wobei insbesondere die Ausbeute an $C_{2+}$-bzw. $C_{3+}$-Kohlenwasserstoffen erhöht werden soll.

Diese Aufgabe wird dadurch gelöst, daß die nach der partiellen Kondensation abgetrennte gasförmige Fraktion einer Rückwaschkolonne zugeführt wird, in der mit bei der Rektifikation gewonnenem Restgas nach dessen teilweiser Kondensation $C_{2+}$- bzw. $C_{3+}$-Kohlenwasserstoffe aus der gasförmigen Fraktion ausgewaschen werden und die im Sumpf der Rückwaschkolonne anfallende flüssige Fraktion der Rektifikation zugeführt wird.

Durch das erfindungsgemäße Verfahren wird eine sehr weitgehende Abtrennung der $C_{2+}$- bzw. $C_{3+}$-Kohlenwasserstoffe in der Rückwaschkolonne erreicht. Dies ist insbesondere deshalb überraschend, weil die Kondensation dieser schweren, noch in der gasförmigen Fraktion enthaltenen Komponenten durch Inkontaktbringen mit einer leichteren Fraktion erreicht wird. Üblicherweise werden dagegen zum Aus-waschen bestimmter Kohlenwasserstoffe aus einem Gas Kohlenwasserstoffe, die schwerer als die auszuwaschenden Komponenten sind, als Waschmittel eingesetzt.

Die durch das erfindungsgemäße Verfahren erreichbare hohe Ausbeute ist auf das günstige Zusammenwirken mehrerer Effekte zurückzuführen. Unter anderem wirkt dabei das partiell kondensierte Restgas der Rektifikation als Kältemittel, da es beim Eintritt in die Rückwaschkolonne vom relativ hohen Partialdruck, unter dem das Kondensat gebildet wurde, auf einen niedrigen Partialdruck entspannt wird, so daß ein Teil des Kondensats unter Freisetzung von Kälte wieder verdampft. Die daraus resultierende Abkühlung führt zu einer Temperatur, die deutlich unter der tiefsten, bei der partiellen Kondensation des Gasstroms herrschenden Temperatur liegt, wodurch weitere, noch in der gasförmigen Fraktion verbliebene schwere Komponenten kondensieren. Da andererseits der in der Rückwaschkolonne erzielbare Abscheidegrad deutlich höher liegt als allein augrund der zusätzlichen Abkühlung zu erwarten wäre, müssen daneben noch andere Effekte, insbesondere wohl Löslichkeitseffekte, eine günstige Rolle spielen.

In einer günstigen Weiterbildung der Erfindung wird die im Sumpf der Rückwaschkolonne anfallende flüssige Fraktion der Rektifikation als Rücklaufflüssigkeit zugeführt. Damit entfällt auch die Notwendigkeit, für die Rektifikation separat, beispielsweise unter Einsatz von Fremdkälte, Rücklaufflüssigkeit zu erzeugen.

In einer weiteren günstigen Weiterbildung der Erfindung wird das Kopfprodukt der Rückwaschkolonne, das im allgemeinen als Restgas nach erneuter Anwärmung auf Umgebungstemperatur abgegeben wird, zunächst entspannt und danach zur Kopfkühlung der Rückwaschkolonne eingesetzt. Die zusätzliche Kopfkühlung der Rückwaschkolonne führt zu einer weiteren, verstärkten Kühlung und damit zu einer weiteren Steigerung der Ausbeute des Verfahrens. Die Entspannung kann durch einfache Drosselung des Gasstroms in einem Ventil erfolgen, bei hinreichend großen Gasströmen oder bei einem hohen. Anteil leichter Komponenten, beispielsweise bei einem Wasserstoffanteil von mehr als 20 %, kann jedoch auch der Einsatz einer Expansionsturbine sinnvoll sein.

Eine weitere besonders günstige Ausgestaltung der Erfindung besteht darin, daß die nach der partiellen Kondensation abgetrennte gasförmige Fraktion, bevor sie in die Rückwaschkolonne geführt wird, zunächst durch einen weiteren indirekten Wärmetausch auf eine tiefere Temperatur abgekühlt wird. Die dabei kondensierenden Bestandteile, die einen relativ großen Teil der in der gasförmigen Fraktion verbliebenen $C_{2+}$- bzw. $C_{3+}$-Kohlenwasserstoffe enthalten, werden abgetrennt und ebenfalls in die Rektifikation geführt, während lediglich der nicht kondensierte Teil der gasförmigen Fraktion der Rückwaschkolonne zugeführt wird. Diese Verfahrensweise bietet insbesondere dann Vorteile, wenn die in der Rückwaschkolonne anfallende Sumpffraktion als Rücklaufflüssigkeit in der Rektifikation eingesetzt wird. Die aus der gasförmigen Fraktion zunächst bevorzugt kondensierenden schweren Bestandteile werden dann nämlich zu einem großen Teil vor der Bildung der Rücklaufflüssigkeit abgetrennt und können der Rektifikation an einer günstigeren Stelle als am Kopf der Rektifiziersäule zugeführt werden.

In günstiger Weiterbildung dieser Verfahrensvariante werden die beim indirekten Wärmetausch aus der gasförmigen Fraktion auskondensierenden Bestandteile gemeinsam mit der bei der partiellen Kondensation gewonnenen flüssigen Fraktion in die Rektifikation geführt. Zwar ist auch die getrennte Führung und Einspeisung beider Kondensatfraktionen möglich, doch sind die dadurch erzielbaren Vorteile häufig so gering, daß sich der apparative Mehraufwand nicht lohnt. Die gemeinsame Verarbeitung beider Kondensatfraktionen kann dagegen besonders einfach gestaltet werden, indem die weitere Abkühlung und partielle Kondensation der gasförmigen Fraktion einfach durchgeführt wird innerhalb eines Abscheiders, der für die Trennung der flüssigen von der gasförmigen Fraktion nach der ersten partiellen Kondensation ohnehin vorzusehen ist. Durch die Anordnung eines Wärmetauschers im oberen Bereich des Abscheiders ergibt sich somit ohne weitere konstruktive Maßnahmen eine Vermischung der beiden Kondensatfraktionen.

Die Abkühlung des Restgases der Rektifikation nebst der dabei erfolgenden partiellen Kondensation erfolgt in typischer Weise bis auf eine Temperatur, bei der 50 bis 99 %, insbesondere 70 bis 95 %, beispielsweise etwa 90 % des Restgases kondensiert sind. Das Restgas besteht bei einer $C_{3+}$-Abtrennung typischerweise aus geringen Mengen Wasserstoff (sofern im Gasstrom Wasserstoff enthalten ist), Methan, $C_2$-Kohlenwasserstoffen als Hauptkomponente und geringen Anteilen $C_3$-Kohlenwasserstoffen und hängt in seiner Zusammensetzung im Einzelfall stark von der speziellen Zusammensetzung des zu zerlegenden Gasstroms und den konkret eingestellten Verfahrensbedingungen ab. Im Fall einer $C_{2+}$-Abtrennung verschiebt sich das Komponentenspektrum zu Methan als Hauptkomponente, während $C_2$ nur noch in geringen Mengen und $C_3$ praktisch überhaupt nicht mehr im Restgas enthalten ist. Um die partielle Kondensation zu bewirken, wird das Restgas mindestens bis auf die Temperatur abgekühlt, auf die auch der Gasstrom im Rahmen seiner partiellen Kondensation abgekühlt wird. Dazu kann der Restgasstrom durch einen separaten Querschnitt des für die Rohgaskühlung eingesetzten Wärmetauschers geführt werden, obwohl auch eine Abkühlung in einem separaten Wärmetauscher in Frage kommt.

Beim Kopf der Rückwaschkolonne stellt sich eine deutlich tiefere Temperatur als die des Gases nach der partiellen Kondensation ein, beispielsweise eine um 10 bis 20 °C tiefere Temperatur. Sofern die aus dem Kopf der Rückwaschkolonne austretende gasförmige Fraktion, die als Restgas angewärmt und aus der Anlage abgezogen wird, direkt durch den für die Rohgaskühlung eingesetzten Wärmetauscher geführt wird, ergeben sich am kalten Ende dieses Wärmetauschers relativ große Temperaturdifferenzen und damit relativ große Wärmeverluste. Um dies zu vermeiden, wird in einer weiteren Ausgestaltung der Erfindung vorgeschlagen, daß mindestens ein Teil des Kopfprodukts der Rückwaschkolonne zunächst in Wärmetausch mit dem bereits teilweise gekühlten bzw. kondensierten Restgas der Rektifikation tritt. Auf diese Weise lassen sich nicht nur große Temperaturdifferenzen am kalten Ende des für die Rohgaskühlung eingesetzten Wärmetauschers vermeiden, sondern als zusätzlicher Effekt ergibt sich dabei eine weitere Abkühlung bzw. weitere Kondensation des Restgases der Rektifikation.

In einer günstigen Weiterbildung der Erfindung wird die nach der partiellen Kondensation aus dem Rohgas abgetrennte flüssige Fraktion vor ihrer Rektifikation mindestens teilweise gegen den abzukühlenden Gasstrom erwärmt und das sich dabei bildende Flüssigkeit-Gas-Gemisch an einer geeigneten Stelle in eine Rektifikationssäule geführt.

Bei der Verarbeitung von Gasströmen, die reich an leichter als Methan siedenden Komponenten sind, ist in Weiterbildung der Erfindung vorgesehen, daß eine Anreicherung dieser Komponenten erfolgen kann, wozu aus dem Kopfprodukt der Rückwaschkolonne $C_1$- und $C_2$-Kohlenwasser-

stoffe durch partielle Kondensation abgetrennt werden. Diese Verfahrensweise kann beispielsweise bei der Abtrennung von C$_{2+}$- bzw. C$_{3+}$-Kohlenwasserstoffen und von Stickstoff aus stickstoffreichem Erdgas oder insbesondere zur Gewinnung der genannten schweren Kohlenwasserstoffe und von Wasserstoff aus wasserstoffreichen Raffineriegasen zum Einsatz kommen. Eine solche Abtrennung ist insbesondere dann von Vorteil, wenn der Einsatzstrom einen relativ hohen Anteil tiefsiedender Komponenten aufweist, beispielsweise einen Wasserstoffgehalt in der Größenordnung von 50 bis 90%. Eine solche Wasserstoffmenge reicht nämlich aus, um die für die zusätzliche Abtrennung benötigte Kälte durch Entspannung zu erzeugen, ohne daß zusätzliche Fremdenergie aufgewendet werden muß.

In vielen Anwendungsfällen ist eine weitere Zerlegung des C$_{2+}$- bzw. C$_{3+}$-Kohlenwasserstoffprodukts, insbesondere eine Trennung zwischen einem C$_3$/C$_4$-Kohlenwasserstoffgemisch und C$_{5+}$-Kohlenwasserstoffen erwünscht. Zu diesem Zweck wird gemäß einer bevorzugten Ausgestaltung des erfindungsgemäßen Verfahrens vor der Bildung der flüssigen und der gasförmigen Fraktion der Großteil der C$_{5+}$-Kohlenwasserstoffe aus dem Gasstrom abgetrennt, sofern die Konzentration dieser Bestandteile so groß ist, daß sich eine derartige Abtrennung lohnt.

Die C$_{5+}$-Abtrennung erfolgt zweckmäßig durch partielle Kondensation bei einer Temperatur oberhalb der Temperatur, bei der die oben erwähnte flüssige und gasförmige Fraktion gebildet werden. Durch die vorherige Abtrennung der schweren Komponenten ist das der Rektifikation zugeführte Gemisch nahezu frei an C$_{5+}$-Kohlenwasserstoffen, so daß bei der nachfolgenden Rektifikation der flüssigen Fraktion ein Produktstrom gewonnen wird, der bei einer C$_{3+}$-Abtrennung eine handelsübliche LPG-Fraktion bildet.

Um die Ausbeute von C$_3$- und C$_4$-Kohlenwasserstoffen zu erhöhen, wird in weiterer Ausbildung dieser Verfahrensvariante vorgeschlagen, daß die abgetrennten schweren Kohlenwasserstoffe ebenfalls der Rektifikation zugeführt werden, wobei die Einspeisung der C$_{5+}$-Fraktion in eine Rektifiziersäule entsprechend dem Gleichgewichtsverlauf in der Rektifziersäule unterhalb der Einspeisung der bei der partiellen Kondensation gebildeten flüssigen Fraktion erfolgt und wobei ferner vorgesehen ist, daß ein im wesentlichen C$_3$- und C$_4$-Kohlenwasserstoffe enthaltender Strom zwischen den beiden Einspeisungen entnommen wird. Durch die zusätzliche Rektifikation der C$_{5+}$-Fraktion werden auch noch die C$_3$/C$_4$-Kohlenwasserstoffe, die bei der Bildung der C$_{5+}$-Fraktion durch partielle Kondensation in Lösung gegangen sind, als Produkt zurückgewonnen. Zwischen den beiden Einspeisestellen bildet sich innerhalb der Rektifiziersäule ein Bereich maximaler C$_3$/C$_4$-Konzentration aus, wo in günstiger Weise der C$_3$/ C$_4$-Produktstrom entnommen wird.

Eine Anlage zur Durchführung des erfindungsgemäßen Verfahrens enthält als wesentliche Bauteile mindestens einen Wärmetauscher zur Abkühlung und partiellen Kondensation des Gasstroms, einen Abscheider zur Abtrennung des partiell kondensierten Teils des Gasstroms und eine Rektifiziersäule zur Zerlegung des partiell kondensierten Teils des Gasstroms sowie eine Rückwaschkolonne, deren unterer Bereich mit dem Dampfraum des Abscheiders und deren oberer Bereich mit dem Kopf der Rektifiziersäule verbunden ist, wobei zwischen dem Kopf der Rektifiziersäule und dem oberen Bereich der Rückwaschkolonne ein Wärmetauscher geschaltet ist.

In einer besonders günstigen konstruktiven Ausgestaltung der Anlage sind der Abscheider und die Rückwaschkolonne in einem gemeinsamen Behälter angeordnet. Dies kann in günstiger Weise so erfolgen, daß die Rückwaschkolonne oberhalb des Abscheiders angeordnet ist und durch einen Kaminboden vom Abscheider getrennt ist, so daß die aus dem Abscheider austretende gasförmige Fraktion über den Kaminboden in den unteren Bereich der Rückwaschkolonne eintreten kann. In weiterer konstruktiver Ausgestaltung der Anlage sind im oberen Bereich des Abscheiders Wärmetauscherrohre angeordnet, durch die kalte Verfahrensströme oder andere Medien geführt werden können, um ein weiteres Auskondensieren schwerer Bestandteile aus der gasförmigen Fraktion vor deren Einleitung in die Rückwaschkolonne zu bewirken.

Weitere Einzelheiten der Erfindung werden nachfolgend anhand der in den Figuren schematisch dargestellten Ausführungsbeispiele erläutert.

Es zeigen :

Figur 1 eine erste Ausführungsform des erfindungsgemäßen Verfahrens,

Figur 2 eine weitere Ausführungsform des erfindungsgemässen Verfahrens, bei dem zusätzlich eine C$_{5+}$-Abtrennung erfolgt,

Figur 3 eine weitere Ausführungsform des erfindungsgemässen Verfahrens, bei dem der Abscheider und die Rückwaschkolonne zu einer baulichen Einheit zusammengefaßt sind und

Figur 4 eine weitere Ausführungsform dieser Verfahrensvariante.

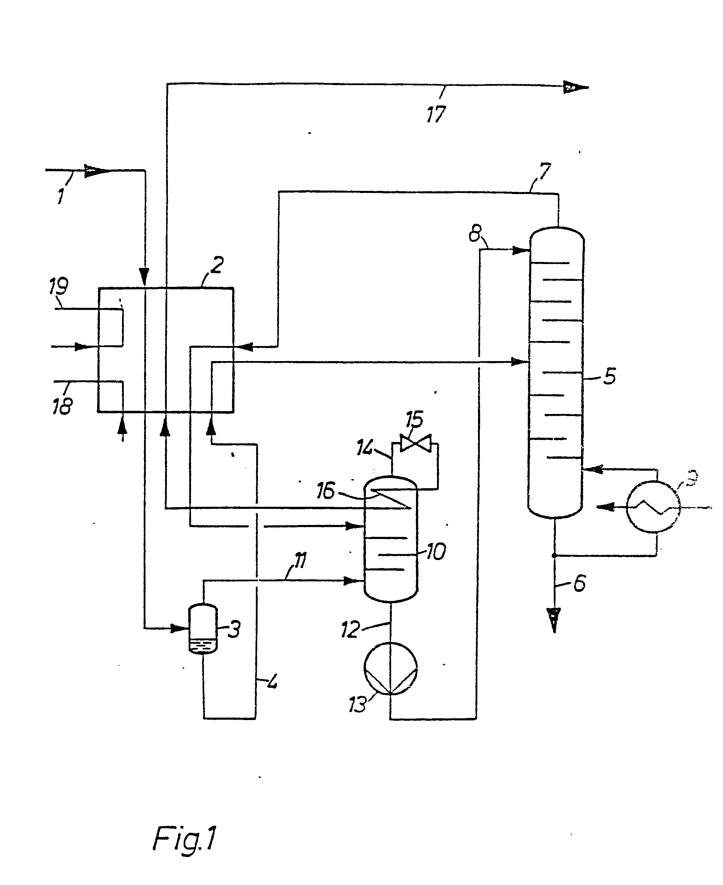

Beim in der Figur 1 dargestellten Ausführungsbeispiel wird über Leitung 1 der zu zerlegende Gasstrom unter erhöhtem Druck und bei annähernd Umgebungstemperatur einem Wärmetauscher 2 zugeführt, in dem er so weit abgekühlt wird, daß der größte Teil der abzutrennenden Kohlenwasserstoffe, also der C$_{2+}$- bzw. C$_{3+}$-Kohlenwasserstoffe kondensiert ist. Der partiell kondensierte Gasstrom wird in einem Abscheider 3 einer Phasentrennung unterzogen, wobei das Kondensat über Leitung 4 zunächst im Wärmetauscher 2 partiell verdampft und dann einer Rektifiziersäule 5 zugeführt wird. In der Rektifiziersäule 5 wird es in eine C$_{2+}$- bzw. C$_{3+}$-Fraktion, die als Produktstrom über Leitung 6 aus dem Sumpf der Säule abgezogen wird, und in einen leichter siedende Anteile enthaltenen Rest-gasstrom 7 zerlegt. Die Rektifikation wird durchgeführt unter Verwendung eines über Leitung 8 herangeführten Fremdrücklaufs sowie einer Sumpfheizung 9, die beispielsweise mit Niederdruckdampf oder mit Heißwasser betrieben wird.

Das über Leitung 7 abgezogene Kopfprodukt der Rektifikation, das im wesentlichen aus Komponenten besteht, die leichter als die über Leitung 6 abgezogene Produktfraktion sieden, wird in den Wärmetauscher 2 geleitet und erneut abgekühlt, wobei noch in diesem Gas verbliebene höhersiedende Komponenten partiell kondensieren. Das dabei gebildete Kondensat fällt in einer Menge an, die größer als die Menge der für die Rektifikation benötigten Rücklaufmenge ist. Dieses partiell kondensierte Restgas wird in den oberen Bereich einer Rückwaschkolonne 10 gegeben, in der es im Gegenstrom mit der aus dem Abscheider 3 über Leitung 11 herangeführten gasförmigen Fraktion in Kontakt gebracht wird. Die im Sumpf der Kolonne 10 anfallende Flüssigkeit wird über Leitung 12 abgezogen und mittels der Pumpe 13 der Rektifiziersäule 5 als Fremdrücklauf über Leitung 8 aufgegeben. Am Kopf der Rückwaschsäule wird über Leitung 14 eine gasförmige Fraktion abgezogen, die nahezu vollständig von den abzutrennenden $C_{2+}$- bzw. $C_{3+}$-Kohlenwasserstoffen befreit ist. Dieser Gasstrom wird im Ventil 15 auf einen gewünschten Restgasdruck entspannt, die dabei gewonnene Kälte wird in einer Kühlfalle 16 an die gasförmige Fraktion in der Rückwaschkolonne 10 übertragen. Anschließend wird das Restgas im Wärmetauscher 2 auf Umgebungstemperatur erwärmt und schließlich über Leitung 17 abgezogen.

In einem konkreten Ausführungsbeispiel gemäß Figur 1 wird über Leitung 1 ein Rohgas bei einer Temperatur von 313 K und unter einem Druck von 20 bar herangeführt. Es enthält 15% Wasserstoff (Prozentangaben nachfolgend immer in Mol-%), 3% Stickstoff, 37 % Methan, 26 % Ethan, 14 % Propan, 4 % Butan und 1 % Pentan. Nach Abkühlung im Wärmetauscher 2 auf 237 K wird im Abscheider 3 ein Kondensat abgetrennt, das 0,4 % Wasserstoff, 0,2 % Stickstoff, 9,4 % Methan, 38,5 % Ethan, 36,3 % Propan, 12,1 % Butan und 3,1 % Pentan enthält. Der gasförmig verbliebene Anteil wird in der Rückwaschkolonne 10 mit ebenfalls auf 237 K abgekühltem Restgas aus der Rektifikation in Kontakt gebracht. Dieses Restgas aus Leitung 7 enthält 0,5 % Wasserstoff, 0,3 % Stickstoff, 15,0 % Methan, 80,8 % Ethan und 3,4 % Propan. Das Kopfprodukt der Rückwaschkolonne wird über Leitung 14 bei einer Temperatur von 221 K abgezogen, im Ventil 15 auf Restgasdruck entspannt, in der Kühlfalle 16 auf 218 K angewärmt und anschließend im Wärmetauscher 2 auf 310 K weiter erwärmt, bevor es über Leitung 17 als Restgas mit einem Druck von 5 bar abgegeben wird. Dieses Restgas enthält 18,6 % Wasserstoff, 3,7 % Stickstoff, 45,8 % Methan, 31,7 % Ethan und nur noch 0,2 % Propan.

Das über Leitung 12 aus dem Sumpf der Rückwaschkolonne 10 abgezogene flüssige Produkt besteht aus 0,3 % Wasserstoff, 0,2 % Stickstoff, 10,5 % Methan, 74,0 % Ethan, 14,2 % Propan und 0,8 % Butan und Pentan. Es wird dem Kopf der bei 18 bar betriebenen Rektifikationssäule 5 zugeführt. Im Sumpf der Rektifikationssäule fällt über Leitung 6 ein $C_{3+}$-Produktstrom an, der 2,0 % Ethan, 71,9 % Propan, 20,9 % Butan und 5,2 % Pentan enthält. Die $C_{3+}$-Ausbeute dieses Verfahrens liegt bei 98,9 %.

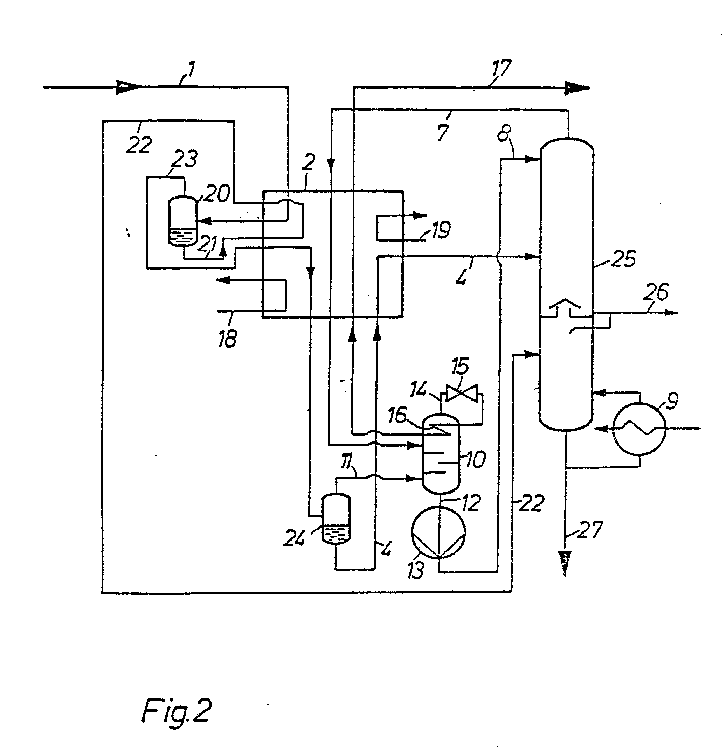

Das in der Figur 2 dargestellte Ausführungsbeispiel zeigt eine Variante des erfindungsgemäßen Verfahrens, bei der in einem ersten Verfahrensschritt eine $C_{5+}$-Abtrennung aus dem Gasgemisch vorgenommen wird. Der Einsatzstrom 1 wird dazu im Wärmetauscher 2 zunächst nur so weit abgekühlt, daß der größte Teil der $C_{5+}$-Komponenten kondensiert. Das teilweise abgekühlte Gemisch wird bei einer Zwischentemperatur aus dem Wärmetauscher 2 herausgeführt, in einem Abscheider 20 einer Phasentrennung unterzogen, wobei die kondensierten Anteile über Leitung 21 abgetrennt, nach teilweiser Erwärmung im Wärmetauscher 2 über Leitung 22 abgezogen und einer Rektifikation zugeführt werden. Der gasförmig verbliebene Anteil des Gasstroms wird über Leitung 23 abgekühlt und schließlich dem Abscheider 24 zugeführt, der dem Abscheider 3 des vorher beschriebenen Ausführungsbeispiels entspricht.

Die Rektifikation der in den Abscheidern 20 und 24 abgetrennten Kondensate erfolgt in einer Trennsäule 25, die gegenüber der im vorausgegangenen Beispiel eingesetzten Trennsäule eine größere Anzahl von Böden aufweist. Zwischen den beiden Zuführungsleitungen 4 und 22 weist diese Säule eine Entnahmeleitung 26 an der Stelle auf, wo sich die höchste $C_{3/4}$-Konzentration befindet. Im Sumpf der Säule 25 fällt eine Flüssigkeit an, die im wesentlichen $C_{5+}$-Kohlenwasserstoffe enthält und die über Leitung 27 als Produktstrom abgezogen wird. Vom Kopf der Säule 25 wird über Leitung 7 wie im vorausgegangenen Beispiel eine leichte Fraktion, die im wesentlichen $C_1$- und $C_2$-Kohlenwasserstoffe enthält, abgezogen.

Bei diesem Verfahren werden die schweren Anteile, die im Abscheider 20 abgetrennt worden sind, ebenfalls der Rektifikation zugeführt. Auf diese Weise läßt sich mit relativ geringem Aufwand eine sehr hohe Ausbeute an $C_3$- und $C_4$-Kohlenwasserstoffen erzielen.

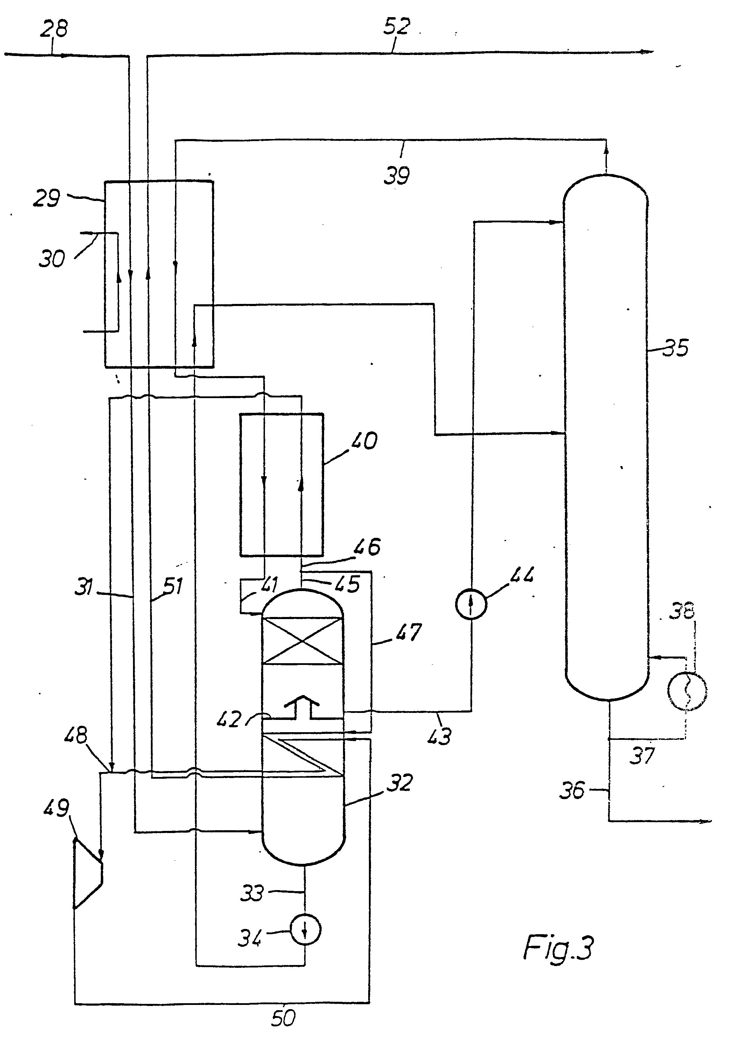

Beim in Figur 3 dargestellten Ausführungsbeispiel wird über Leitung 28 ein Rohgas bei einer Temperatur von 305 K und einem Druck von 28,9 bar herangeführt. Es enthält 67,5 % Wasserstoff, 11,8 % Methan, 8,8 % $C_2$-, 7,8 % $C_3$-, 3,3 % $C_4$- und 0,8 % $C_{5+}$-Kohlenwasserstoffe. Nach Abkühlung im Wärmetauscher 29 auf eine Temperatur von 230 K, die durch indirekten Wärmetausch mit anzuwärmenden Verfahrensströmen und mit einem durch Leitung 30 schematisch angedeuteten Kältekreislauf bewirkt wird, wird das partiell kondensierte Gemisch über Leitung 31 einem Abscheider zugeführt, der im unteren Teil eines Behälters 32 angeordnet ist, dessen oberer Teil die Rückwaschkolonne bildet. Aus dem Abscheider wird über Leitung 33 eine Flüssigkeit abgezogen, die 1,4 % Wasserstoff, 3,5 % Methan, 23,0 %

$C_2$-, 45,1 % $C_3$-, 21,5 % $C_4$- und 5,5 % $C_{5+}$-Kohlenwasserstoffe enthält. Die Sumpfflüssigkeit wird mittels der Pumpe 34 bei einem Druck von 30 bar zunächst dem Wärmetauscher 29 zugeführt und nach teilweiser Anwärmung in die Rektifiziersäule 35 eingespeist. In der Rektifiziersäule 35 wird es in eine $C_{3+}$-Sumpffraktion und eine $C_2$-Fraktion zerlegt. Die $C_{3+}$-Kohlenwasserstoffe werden am Sumpf über Leitung 36 als Produktstrom abgezogen. Ein Teilstrom wird über Leitung 37 abgezweigt, im Aufkocher 38 erwärmt und als Sumpfheizung in den unteren Bereich der Kolonne 35 zurückgeführt. Das Produkt in Leitung 36 fällt bei einer Temperatur von 362 K und einem Druck von 29 bar an und besteht aus 2,0 % $C_2$-, 63,6 % $C_3$-, 27,5 % $C_4$- und 6,9 % $C_{5+}$-Kohlenwasserstoffen. Am Kopf der Rektifiziersäule 35 fällt eine Fraktion an, die 2,8 % Wasserstoff, 8,7 % Methan, 84,4 % $C_2$- und 4,1 % $C_3$-Kohlenwasserstoffe enthält. Diese Fraktion wird über Leitung 39 abgezogen und vor Einspeisung in die Rückwaschkolonne abgekühlt. Die Abkühlung erfolgt zunächst im Wärmetauscher 29 auf eine Temperatur von etwa 230 K und anschließend im Wärmetauscher 40 auf eine Temperatur von 202 K. Anschließend wird die größtenteils kondensierte Fraktion über Leitung 41 auf den Kopf der Rückwaschkolonne im oberen Teil des Behälters 32 aufgegeben. Im unteren Bereich der Rückwaschkolonne sammelt sich auf dem Kaminboden 42, der die Rückwaschkolonne vom darunter gelegenen Abscheider trennt, eine Flüssigkeit. Diese besteht einerseits aus dem Kopfprodukt der Rektifikation, so weit es in den Wärmetauschern 29, 40 kondensiert und in der Rückwaschkolonne nicht wieder verdampft ist und andererseits aus den ausgewaschenen schweren Komponenten, die aus der im Abscheider anfallenden gasförmigen Fraktion ausgewaschen wurden. Die gasförmige Fraktion aus dem Abscheider gelangt über den Kaminboden 42 direkt in den unteren Teil der Rückwaschkolonne. Die sich auf dem Kaminboden 42 ansammelnde Flüssigkeit wird über Leitung 43 abgezogen und mittels der Pumpe 44 als Rücklaufflüssigkeit auf den Kopf der Rektifiziersäule 35 aufgegeben. Diese Rücklaufflüssigkeit fällt bei einer Temperatur von 200 K an und besteht aus 1,5 % Wasserstoff, 6,0 % Methan, 78,6 % $C_2$-, 13,6 % $C_3$- und 0,3 % $C_4$-Kohlenwasserstoffen.

In der Rückwaschkolonne fällt am Kopf ein über Leitung 45 abgezogenes Restgas an, das in zwei Teilströme aufgeteilt wird. Ein erster Teilstrom gelangt über Leitung 46 in den Wärmetauscher 40, wo es zur Kühlung der Kopffraktion aus der Rektifikation von etwa 230 K auf 202 K herangezogen wird, während der andere Teilstrom über Leitung 47 in den oberen Bereich des Abscheiders geführt wird, um hier seine Spitzenkälte an die gasförmige Fraktion im Abscheider abzugeben. Nach partieller Anwärmung im Abscheider werden die über die Leitungen 46 und 47 verzweigten Teilströme bei 48 wieder vereinigt und in einer Turbine 49 arbeitsleistend soweit entspannt, daß der einzuhaltende Abgabedruck für das Restgas am Austrittsende der Anlage eingehalten wird. Das bei der Entspannung abgekühlte Restgas wird über Leitung 50 zunächst noch einmal durch den Dampfraum des Abscheiders geführt und gelangt anschließend über Leitung 51 zum Wärmetauscher 29, in dem es sich gegen abzukühlende Verfahrensströme auf eine Temperatur von 302 K anwärmt, bevor es über Leitung 52 bei einem Druck von 17 bar abgezogen wird. Dieses Gas enthält 76,8 % Wasserstoff, 13,3 % Methan, 9,8 % $C_2$- und 0,1 % $C_3$-Kohlenwasserstoffe.

Bei diesem Verfahren fallen 99,5 % der im Rohgas enthaltenen $C_{3+}$-Kohlenwasserstoffe im Sumpf der Rektifiziersäule 35 an und werden über Leitung 36 als Produkt abgezogen.

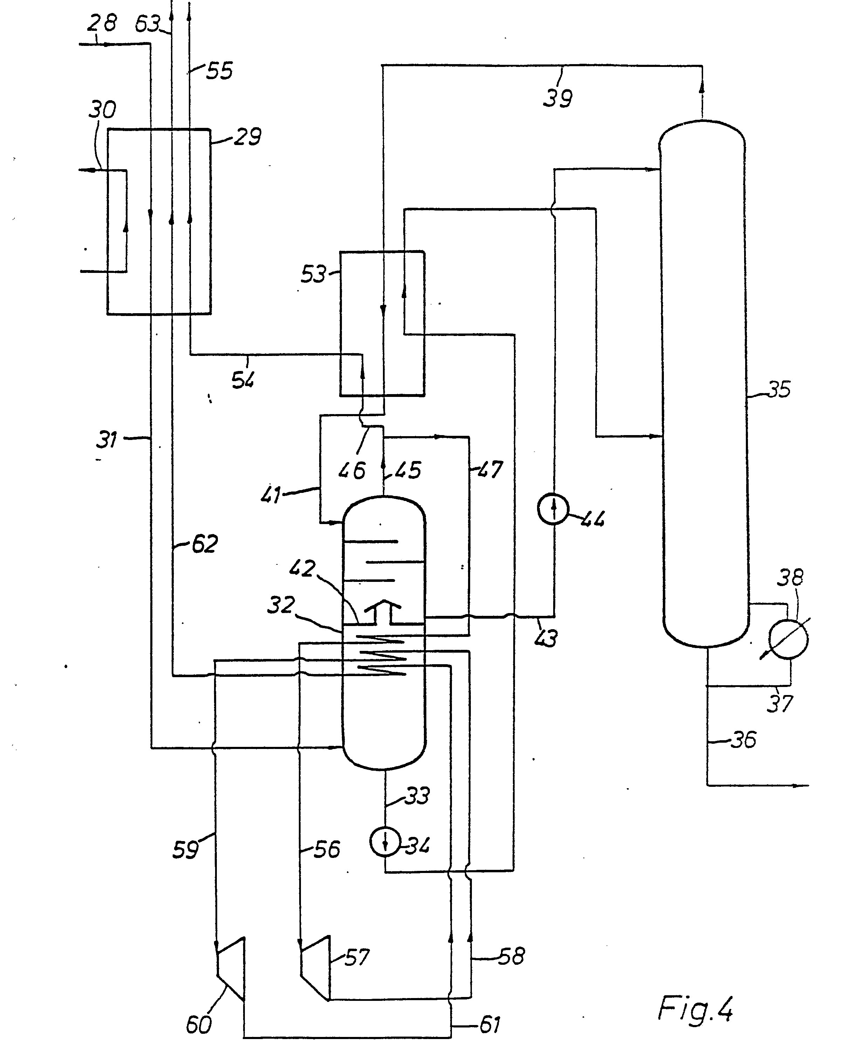

Das in Figur 4 dargestellte Verfahren stellt eine Abwandlung des in Figur 3 dargestellten und zuvor beschriebenen Verfahrens dar. Deshalb seien nur die Verfahrensunterschiede angegeben. Gemäß Figur 4 wird das Kopfprodukt der Rektifiziersäule 35 über Leitung 39 einem Wärmetauscher 53 zugeführt, in dem es zunächst gegen Sumpfprodukt aus dem Abscheider, das vor Einspeisung in die Rektifiziersäule 35 angewärmt wird, und dann gegen Kopfprodukt aus der Rückwaschkolonne, das über Leitung 46 abgezweigt wird, abgekühlt wird. Der Teilstrom 46 des Kopfprodukts der Rückwaschkolonne wird nach Austritt aus dem Wärmetauscher 53 über Leitung 54 unmittelbar dem Wärmetauscher 29 zugeleitet und auf Umgebungstemperatur angewärmt. Hierdurch fällt ein über Leitung 55 abgezogener Teil des Restgase beim Druck der Rückwaschkolonne, der lediglich durch die unvermeidlichen Druckverluste in den Leitungen und Wärmetauschern vermindert ist, an. Der über Leitung 47 abgezweigte Teilstrom des Kopfprodukts der Rückwaschkolonne wird zunächst wieder im oberen Bereich des Abscheiders abgekühlt und danach über Leitung 56 einer ersten Expansionsturbine 57 zugeführt, und auf einen mittleren Druck entspannt. Die dabei gewonnene Kälte wird ebenfalls auf die Dampffraktion im Abscheider übertragen, wozu über Leitung 58 der Turbinenaustritt erneut durch einen Wärmetauscher im oberen Bereich des Abscheiders geleitet wird. Das austretende Gas wird über Leitung 59 einer zweiten Expansionsturbine 60 zugeführt und erneut unter Abkühlung arbeitsleistend entspannt. Über Leitung 61 wird das Restgas erneut in den oberen Bereich des Abscheiders geleitet und gibt nochmals seine Spitzenkälte an die Dampffraktion im Abscheider ab, bevor es schließlich über Leitung 62 zum Wärmetauscher 29 geleitet wird und über Leitung 63 als Niederdruck-Restgas abgezogen wird.

Die Rektifikationsbedingungen, insbesondere Druck und Temperatur, bei der Gewinnung der $C_2$- bzw. der $C_{3+}$-Kohlenwasserstoffe werden üblicherweise aufgrund verschiedener Randbedingungen, insbesondere unter Berücksichtigung der Zusammensetzung des zu rektifizierenden Gemisches, abgestimmt. Weiterhin kann auch das zu zerlegende Gasgemisch unter verschiedenen Bedingungen, insbesondere unter unterschiedlich hohem Druck, bereitstehen. Im Einzelfall kann sich daraus ergeben, daß das Trennverfahren unter optimalen Bedingungen so durchgeführt wird, daß der Druck in der Rektifikation höher oder auch niedriger als der Druck des partiell kondensierten Gasstroms ist. Bei den Ausführungsbeispielen gemäß den Figuren 1 bis 4 wurde angenommen, daß keine wesentlichen Druckdifferenzen bestehen. Sollten solche jedoch im Einzelfall bestehen, kann die beschriebene Verfahrensführung den veränderten Bedingungen in einfacher Weise angepaßt werden, beispielsweise im Fall einer Rektifikation unter höherem Druck durch Anordnung einer Pumpe in Leitung 4, Ersatz der Förderpumpe 13 durch eine Pumpe mit entsprechend höherem Verdichtungsverhältnis und durch Entspannung des partiell kondensierten Restgases in die Rückwaschkolonne 10. (Figuren 1 und 2).

## Patentansprüche

1. Verfahren zur Abtrennung von $C_{2+}$- oder von $C_{3+}$-Kohlenwasserstoffen aus einem leichte Kohlenwasserstoffe und gegebenenfalls leichter als Methan siedende Komponenten enthaltenden Gasstrom, bei dem der unter erhöhtem Druck stehende Gasstrom abgekühlt, partiell kondensiert und in eine flüssige und eine gasförmige Fraktion getrennt wird und bei dem die flüssige Fraktion durch Rektifikation in einen im wesentlichen $C_{2+}$- bzw. $C_{3+}$-Kohlenwasserstoffe enthaltenden Produktstrom und einen überwiegend leichtersiedende Komponenten enthaltenden Restgasstrom zerlegt wird, dadurch gekennzeichnet, daß die nach der partiellen Kondensation (3, 20/24, 32) abgetrennte gasförmige Fraktion (11) einer Rückwaschkolonne (10, 32) zugeführt wird, in der mit bei der Rektifikation gewonnenem Restgas (7, 39) nach dessen teilweiser Kondensation $C_{2+}$- bzw. $C_{3+}$-Kohlenwasserstoffe aus der gasförmigen Fraktion ausgewaschen werden und die im Sumpf (12, 43) der Rückwaschkolonne anfallende flüssige Fraktion der Rektifikation (5, 25, 35) zugeführt wird.

2. Verfahren nach Anspruch 1, dadurch gekennzeichnet, daß die im Sumpf der Rückwaschkolonne anfallende flüssige Fraktion der Rektifikation als Rücklaufflüssigkeit (8, 43) zugeführt wird.

3. Verfahren nach Anspruch 1 oder 2, dadurch gekennzeichnet, daß das Kopfprodukt (14) der Rückwaschkolonne entspannt (15) und danach zur Kopfkühlung (16) der Rückwaschkolonne eingesetzt wird.

4. Verfahren nach einem der Ansprüche 1 bis 3, dadurch gekennzeichnet, daß die partielle Kondensation des Gasstroms (1) sowie des Restgases (7) durch Abkühlung auf das gleiche Temperaturniveau erreicht werden.

5. Verfahren nach einem der Ansprüche 1 bis 4, dadurch gekennzeichnet, daß die nach der partiellen Kondensation abgetrennte gasförmige Fraktion (23, 32) zunächst durch indirekten Wärmetausch (2, 47/50, 47/58/61) mit kälteren Verfahrensströmen gekühlt wird und dabei kondensierende Bestandteile (4, 33) abgetrennt und in die Rektifikation (25, 35) geführt werden und der nicht kondensierte Teil (11, 42) der gasförmigen Fraktion der Rückwaschkolonne zugeführt wird.

6. Verfahren nach Anspruch 5, dadurch gekennzeichnet, daß die beim indirekten Wärmetausch aus der gasförmigen Fraktion auskondensierenden Bestandteile gemeinsam mit der bei der partiellen Kondensation gewonnenen flüssigen Fraktion in die Rektifikation geführt werden.

7. Verfahren nach Anspruch 6, dadurch gekennzeichnet, daß der indirekte Wärmetausch im oberen Bereich eines für die Trennung der flüssigen und gasförmigen Fraktion vorgesehenen Abscheiders erfolgt.

8. Verfahren nach einem der Ansprüche 1 bis 7, dadurch gekennzeichnet, daß das bei der Rektifikation gewonnene Restgas vor dessen Einspeisung in die Rückwaschkolonne zu 50 bis 99 %, Vorzugsweise zu 70 bis 95 % verflüssigt wird.

9. Verfahren nach Anspruch 8, dadurch gekennzeichnet, daß das Restgas (39) in einer letzten Wärmetauschstufe (40, 53) durch indirekten Wärmetausch mit aus der Rückwaschkolonne austretender gasförmiger Fraktion (46) weitgehend verflüssigt wird.

10. Verfahren nach einem der Ansprüche 1 bis 9, dadurch gekennzeichnet, daß die flüssige Fraktion vor der Rektifikation mindestens teilweise gegen den abzukühlenden Gasstrom erwärmt wird.

11. Verfahren nach einem der Ansprüche 1 bis 10, dadurch gekennzeichnet, daß bei der Verarbeitung eines Gasstroms, der reich an leichter als Methan siedenden Komponenten ist, vor der Entspannung des Kopfprodukts der Rückwaschkolonne $C_1$- und $C_2$-Kohlenwasserstoffe durch partielle Kondensation aus dieser Fraktion abgetrennt werden.

12. Verfahren nach einem der Ansprüche 1 bis 11, dadurch gekennzeichnet, daß vor der Bildung der flüssigen und der gasförmigen Fraktion ein Großteil von gegebenenfalls im Gasstrom enthaltenen $C_{5+}$-Kohlenwasserstoffen abgetrennt wird (20).

13. Verfahren nach Anspruch 12, dadurch gekennzeichnet, daß die abgetrennten $C_{5+}$-Kohlenwasserstoffe (21) ebenfalls der Rektifikation zugeführt werden, daß die Einspeisung in eine Rektifiziersäule (22) unterhalb der Einspeisung der bei der partiellen Kondensation gebildeten flüssigen Fraktion (4) erfolgt und daß ein im wesentlichen $C_3$- und $C_4$-Kohlenwasserstoffe enthaltender Strom (26) zwischen den beiden Einspeisungen entnommen wird.

14. Anlage zur Durchführung des Verfahrens nach einem der Ansprüche 1 bis 13 mit mindestens einem Wärmetauscher zur Abkühlung und partiellen Kondensation des Gasstroms, mit einem Abscheider zur Abtrennung des partiell kondensierten Teils des Gasstroms und mit einer Rektifiziersäule zur Zerlegung des partiell kondensierten Teils des Gasstroms, gekennzeichnet durch eine Rückwaschkolonne (10, 32), deren unterer Bereich mit dem Dampfraum des Abscheiders verbunden (11, 42) und deren oberer Bereich mit dem Kopf der Rektifiziersäule verbunden ist (7, 39), wobei zwischen dem Kopf der Rektifiziersäule und dem oberen Bereich der Rückwaschkolonne ein Wärmetauscher geschaltet ist.

15. Anlage nach Anspruch 14, dadurch gekennzeichnet, daß der Abscheider und die Rückwaschkolonne in einem gemeinsamen Behälter (32) angeordnet sind.

16. Anlage nach Anspruch 15, dadurch gekennzeichnet, daß die Rückwaschkolonne oberhalb des Abscheiders angeordnet und durch einen Kaminboden (42) vom Abscheider getrennt ist.

17. Anlage nach einem der Ansprüche 14 bis 16, dadurch gekennzeichnet, daß im oberen Bereich des Abscheiders Wärmetauscherrohre angeordnet sind (Figur 3 und 4).

## Claims

1. A process for separating $C_{2+}$ or $C_{3+}$-hydrocarbons from a gas stream which contains light hydrocarbons and possibly components which boil more easily than methane, wherein the gas stream under increased pressure, is cooled, partially condensed and separated into a liquid fraction and a gaseous fraction, and wherein the liquid fraction is separated by rectification into a product stream which essentially contains $C_{2+}$ or $C_{3+}$-hydrocarbons respectively and a residual gas stream which contains mainly more easily boiling components, characterised in that the gaseous fraction (11) which is separated after the partial condensation (3, 20/24, 32), is fed to a counterflow washing column (10, 32) in which $C_{2+}$ or $C_{3+}$-hydrocarbons respectively are washed out of the gaseous fraction with residual gas (7, 39) obtained during the rectification after its partial condensation, and the liquid fraction accumulating in the sump (12, 43) of the counterflow washing column is fed to the rectification stage (5, 25, 35).

2. A process as claimed in Claim 1, characterised in that the liquid fraction which accumulates in the sump of the counterflow washing column is fed to the rectification stage as reflux liquid (8, 43).

3. A process as claimed in Claim 1 or 2, characterised in that the head product (14) from the counterflow washing column is expanded (15) and is then used for cooling the head (16) of the counterflow washing column.

4. A process as claimed in one of Claims 1 to 3, characterised in that the partial condensation of the gas stream (1) and of the residual gas (7) is achieved by cooling to the same temperature level.

5. A process as claimed in one of Claims 1 to 4, characterised in that the gaseous fraction (23, 32) which is separated after the partial condensation is first cooled by indirect heat exchange (2, 47/50, 47/58/61) with cooler process streams, and components (4, 33) thereby condensed are separated and fed to the rectification stage (25, 35) and the uncondensed part (11, 42) of the gaseous fraction is fed to the counter flow washing column.

6. A process as claimed in Claim 5, characterised in that the components which condense out of the gaseous fraction during the indirect heat exchange are fed to the rectification stage, together with the liquid fraction obtained during the partial condensation.

7. A process as claimed in Claim 6, characterised in that the indirect heat exchange is carried out in the upper region of a separator provided for separating the liquid and gaseous fractions.

8. A process as claimed in one of Claims 1 to 7, characterised in that the residual gas obtained by the rectification is liquefied by 50 to 99 %, preferably 70 to 95 %, before it is fed into the counterflow washing column.

9. A process as claimed in Claim 8, characterised in that the residual gas (39) is liquefied to a large extent in a last heat exchanger stage (40, 53) by indirect heat exchange with the gaseous fraction (46) leaving the counterflow washing column.

10. A process as claimed in one of Claims 1 to 9, characterised in that before the rectification, the liquid fraction is at least partially heated against the gas stream to be cooled.

11. A process as claimed in one of Claims 1 to 10, characterised in that in the treatment of a gas stream which is rich in components which boil more easily than methane, prior to the expansion of the head product from the counterflow washing column, $C_1$ and $C_2$-hydrocarbons are separated from this fraction by partial condensation.

12. A process as claimed in one of Claims 1 to 11, characterised in that prior to the formation of the liquid and gaseous fractions, a large part of the $C_{5+}$-hydrocarbons possibly contained in the stream are separated (20).

13. A process as claimed in Claim 12, characterised in that the separated $C_{5+}$-hydrocarbons (21) are also fed to the rectification stage, that they are introduced into a rectification column (22) beneath the input point of the liquid fraction (4) which is formed during the partial condensation, and that a stream (26) which essentially contains $C_3$ and $C_4$-hydrocarbons is withdrawn between the two input points.

14. Apparatus for carrying out the process claimed in one of Claims 1 to 13 comprising at least one heat exchanger for the cooling and partial condensation of the gas stream, a separator for the separation of the partially condensed part of the gas stream, and a rectification column for separating the partially condensed part of the gas flow, characterised by a counterflow washing column (10, 32), the lower region of which is connected to the vapour space of the separator (11, 42) and the upper region of which is connected to the head of the rectification column (7, 39), a heat exchanger being connected between the head of the rectification column and the upper region of the counterflow washing column.

15. Apparatus as claimed in Claim 14, characterised in that the separator and the counterflow washing column are arranged in a common container (32).

16. Apparatus as claimed in Claim 15, characterised in that the counterflow washing column is arranged above the separator and is separated from the separator by a chimney plate (42).

17. Apparatus as claimed in one of Claims 14 to 16, characterised in that heat exchanger pipes are arranged in the upper region of the separator. (Fig. 3 and 4).

## Revendications

1. Procédé de séparation d'hydrocarbures en $C_{2+}$ ou en $C_{3+}$ à partir d'un courant gazeux contenant des hydrocarbures légers et éventuellement des composants à point d'ébullition plus bas que celui du méthane, selon lequel le courant gazeux à pression élevée est refroidi, partiellement condensé et séparé en une fraction liquide et en une fraction gazeuse et selon lequel la fraction liquide est séparée par rectification en un courant de produit contenant essentiellement des hydrocarbures en $C_{2+}$ et/ou en $C_{3+}$ et en un courant de gaz résiduaire contenant surtout des composants à plus bas point d'ébullition, caractérisé en ce que la fraction gazeuse (11) séparée après la condensation partielle (3, 20/24, 32) est amenée à une colonne de lavage à contre-courant (10, 32) dans laquelle les hydrocarbures en $C_{2+}$ et/ou en $C_{3+}$ sont extraits par lavage de la fraction gazeuse au moyen du gaz résiduaire (7, 39) obtenu dans la rectification après sa condensation partielle, et la fraction liquide produite en cuve (12, 43) de la colonne de lavage à contre-courant est amenée à la rectification (5, 25, 35).

2. Procédé selon la revendication 1, caractérisé en ce que la fraction liquide produite en cuve de la colonne de lavage à contre-courant est amenée à la rectification comme liquide (8, 43) de reflux.

3. Procédé selon la revendication 1 ou 2, caractérisé en ce que le produit (14) de tête de la colonne de lavage à contre-courant est détendu (15) et est ensuite utilisé pour le refroidissement de tête (16) de la colonne de lavage à contrecourant.

4. Procédé selon l'une quelconque des revendications 1 à 3, caractérisé en ce que la condensation partielle du courant de gaz (1) ainsi que du gaz résiduaire (7) est obtenue par refroidissement au même niveau de température.

5. Procédé selon l'une quelconque des revendications 1 à 4, caractérisé en ce que la fraction gazeuse (23, 32) séparée après condensation partielle est tout d'abord refroidie par échange de chaleur indirect (2, 47/50, 47/58/61) avec des courants plus froids et que les constituants (4, 33) qui se condensent alors sont séparés et amenés à la rectification (25, 35), la partie non condensée (11, 42) étant amenée à la fraction gazeuse de la colonne de lavage à contre-courant.

6. Procédé selon la revendication 5, caractérisé en ce que les constituants se séparant par condensation lors de l'échange de chaleur indirect dans la fraction gazeuse passent à la rectification en même temps que la fraction liquide obtenue lors de la condensation partielle.

7. Procédé selon la revendication 6, caractérisé en ce que l'échange de chaleur indirect a lieu dans la partie supérieure d'un séparateur prévu pour la séparation de la fraction liquide et de la fraction gazeuse.

8. Procédé selon l'une quelconque des revendications 1 à 7, caractérisé en ce que le gaz résiduaire obtenu lors de la rectification est liquéfié avant son introduction dans la colonne de lavage à contre-courant à concurrence de 50 à 99 %, de préférence de 70 à 95 %.

9. Procédé selon la revendication 8, caractérisé en ce que le gaz résiduaire (39) est liquéfié dans une large mesure dans une dernière étape d'échange de chaleur (40, 53) par échange de chaleur indirect avec la fraction gazeuse (46) quittant la colonne de lavage à contre-courant.

10. Procédé selon l'une quelconque des revendications 1 à 9, caractérisé en ce que la fraction liquide est réchauffée avant rectification au moins partiellement en échange de chaleur avec le courant de gaz à refroidir.

11. Procédé selon l'une quelconque des revendications 1 à 10, caractérisé en ce que lors du traitement d'un courant de gaz riche en composants de point d'ébullition plus bas que celui du méthane, avant la détente du produit de tête de la colonne de lavage à contre-courant, les hydrocarbures en $C_1$ et en $C_2$ sont séparés de cette fraction par condensation partielle.

12. Procédé selon l'une quelconque des revendications 1 à 11, caractérisé en ce que, avant la formation de la fraction liquide et de la fraction gazeuse, on sépare (20) une grande partie des hydrocarbures en $C_{5+}$ éventuellement contenus dans le courant de gaz.

13. Procédé selon la revendication 12, caractérisé en ce que les hydrocarbures en $C_{5+}$ séparés (21) sont dirigés également de même à la rectification, que l'introduction dans une colonne de rectification (22) se fait en dessous du point d'introduction de la fraction liquide (4) formée lors de la condensation partielle et qu'un courant (26) contenant essentiellement des hydrocarbures en $C_3$ et en $C_4$ est prélevé entre les deux points d'introduction.

14. Installation de mise en oeuvre du procédé selon l'une quelconque des revendications 1 à 13, comportant au moins un échangeur de chaleur pour le refroidissement et la condensation partielle du courant de gaz, à l'aide d'un séparateur pour le fractionnement de la partie partiellement condensée du courant de gaz et à l'aide d'une colonne de rectification pour la séparation de la partie partiellement condensée du courant de gaz, caractérisée par une colonne de lavage à contre-courant (10, 32) dont la partie inférieure communique (11, 42) avec l'espace vapeur du séparateur et sa partie supérieure communique (7, 39) avec la tête de la colonne de rectification, un échangeur de chaleur étant intercalé entre la tête de la colonne de rectification et la partie supérieure de la colonne de lavage à contre-courant.

15. Installation selon la revendication 14, caractérisée en ce que le séparateur et la colonne de lavage à contre-courant sont disposés dans un récipient commun (32).

16. Installation selon la revendication 15, caractérisée en ce que la colonne de lavage à contre-courant est disposée au-dessus du séparateur et isolée de ce séparateur par un plateau à calottes (42).

17. Installation selon l'une quelconque des revendications 14 à 16, caractérisée en ce que des tubes d'échange de chaleur sont disposés dans la partie supérieure du séparateur (figure 3 et 4).

Fig.1

Fig.2

Fig.3

0 185 253

Fig.4